# EUROPEAN PATENT APPLICATION

(11) **EP 4 620 412 A1**
(43) Date of publication of application: **24.09.2025**
(21) Application number: 23890936.0
(22) Date of filing: 14.11.2023
(51) Int. Cl.: A61B 17/435, A61D 19/04, A61M 25/08

(54) **DEVICE FOR INTRODUCING A FERTILISED EGG OR EMBRYO**

(30) Priority: 14.11.2022 ES 202231892 U
(71) Applicant: Premium Fertility SL, 46980 Paterna (Valencia) (ES)
(72) Inventor: SANTAMARIA COSTA, Xavier, 46980 Paterna (Valencia) (ES); SIMÓN VALLÉS, Carlos, 46980 Paterna (Valencia) (ES); FREITAS, Rhys, San Diego, California 92127 (US); BADAL REGAS, Ari, San Diego, California 92127 (US); HUNT, David S., San Diego, California 92127 (US)
(74) Representative: Hoffmann Eitle
(86) International application number: PCT/ES2023/070678
(87) International publication number: WO 2024/105294

(57) **Abstract**

The present invention relates to an apparatus suitable for delivering a fertilized egg or embryo into the maternal uterine endometrium. The apparatus comprises an elongated body comprising a distal tip and a lumen extending therethrough to an aperture at the distal tip. The lumen is configured to receive and expel the fertilized egg or embryo through the aperture. A plunger is configured to be slidably received in the lumen. The distal tip comprises a piercing portion for piercing the maternal uterine endometrium, and the aperture has a concave shape.

## Description

### TECHNICAL FIELD

The present invention relates to the field of apparatus for delivering a fertilized egg or embryo into a maternal uterine endometrium, and specifically apparatus configured to pierce the maternal uterine endometrium and deliver the fertilized egg or embryo.

### BACKGROUND

Human *in vitro* fertilization (IVF) and embryo transfer (ET), successfully performed for the first time in 1978, have become a widely practiced procedure to treat infertile couples who have not achieved success with more conventional therapeutic procedures such as superovulation and intrauterine insemination. The most common indications for IVF and related procedures, such as gamete *in vitro* fertilization or gamete intrafallopian transfer (GIFT), include women having blocked or damaged fallopian tubes, as well as low sperm and/or egg quality. Related factors include the age of the female and the degree of endometrial receptivity. The procedure can also be used in cases of severe male factor infertility in which direct (intracytoplasmic) injection of sperm is an option.

The IVF/ET procedure typically involves the hormonal stimulation of the female to first suppress her ability to ovulate on her own, and then stimulate the development of follicles in the ovaries with a fertility medication. Mature eggs are removed from the ovary transvaginally by means of a needle, preferably guided by ultrasound. After harvesting the eggs, they are identified and sorted based on their maturity, and then placed with a sperm sample from the male. Approximately 24 hours after fertilization, the eggs are examined to confirm fertilization, which occurs in approximately 65% to 85% of the eggs harvested.

After a short development period, the embryos are transferred, along with a volume of fluid, to the uterus by means of a delivery catheter. To increase the chances of success of the procedure, the embryo must be implanted in the uterine endometrium using a needle tip to penetrate the endometrium. Pressure is applied on the fluid in the needle tip so that the embryo can be expelled onto the endometrium.

It has been observed that the embryo may adhere to the distal tip of the needle in a position in which the fluid may flow out of the needle and elude the embryo. In such cases, since the expelled fluid may elude the embryo, the embryo may remain attached to the needle tip even after all of the fluid has been expelled. This may result in the embryo moving or even becoming detached from the endometrium when the needle is withdrawn.

Therefore, there is a need to provide improved apparatus for delivering embryos or fertilized eggs into the uterine endometrium that more effectively expels the embryo or fertilized egg from the apparatus.

### SUMMARY OF THE INVENTION

A first aspect of the invention provides an apparatus suitable for delivering a fertilized egg or embryo into a maternal uterine endometrium, comprising: an elongated body comprising a distal tip and a lumen extending therethrough to an aperture at the distal tip, the lumen being configured to receive and expel the fertilized egg or embryo through the aperture; and a plunger configured to be slidably received in the lumen. The distal tip comprises a piercing portion for piercing the maternal uterine endometrium, and the aperture has a concave shape.

### BRIEF DESCRIPTION OF THE DRAWINGS

To allow a better understanding of the present disclosure, and to show how the same can be carried out, reference will now be made, by way of example only, to the attached schematic drawings, in which:
Figure 1 shows a perspective view of an apparatus according to the prior art.
Figure 2 shows a schematic perspective view of an apparatus according to one or more embodiments.
Figures 3A and 3B show schematic side views of an apparatus according to one or more embodiments.
Figure 4 shows a schematic side view of an apparatus according to one or more embodiments.
Figure 5A shows a schematic top view of an apparatus according to one or more embodiments.
Figure 5B shows a cross-sectional side view of the apparatus shown in Figure 5A taken along line A-A.
Figure 6 shows a schematic view of an apparatus according to one or more embodiments.
Figure 7 shows a schematic view of an apparatus according to one or more embodiments.
Figure 8 shows a schematic view of an apparatus according to one or more embodiments.

### DETAILED DESCRIPTION

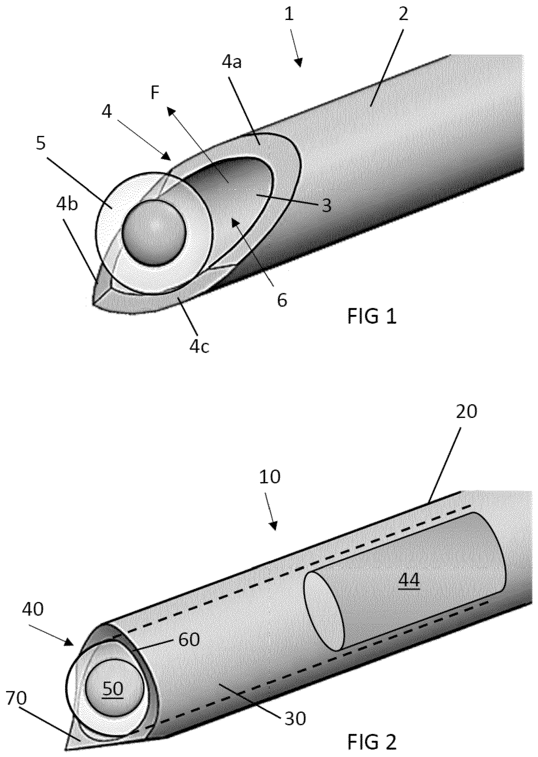

Figure 1 shows a perspective view of an apparatus 1 according to the prior art. The apparatus 1 comprises an elongated body 2 (for example, a needle) comprising a distal tip 4 and a lumen 3 extending therethrough to an aperture 6 at the distal tip 4. The lumen 3 is configured to receive and expel a fertilized egg or embryo 5 (referred to hereinafter as "egg" for short) through the aperture 6. A plunger (not shown) is slidably received in the lumen 3. The distal tip 4 is configured to pierce the maternal uterine endometrium and comprises a first surface 4a which is bevelled with respect to the longitudinal axis of the elongated body 2. The distal tip 4 further comprises a piercing portion at its most distal end, which is a double-bevelled piercing portion comprising a first surface 4b and a second surface 4c which are bevelled with respect to the first surface 4a to form a sharp tip at the distal end of the distal tip 4. In use, the plunger is retractable in a proximal direction opposite the distal tip 4, which creates suction at the distal tip 4 and can be used to place the egg 5 in the lumen 3. The egg 5 is usually placed in the lumen with fluid proximal to the egg 5 in the lumen 3. In order to implant the egg 5 into the endometrium, the elongated body 2 penetrates the endometrium using the distal piercing tip and the plunger is advanced distally to expel the fluid and the egg 5 in the lumen 3. The egg 5 is generally pushed distally due to the pressure transmitted by the plunger through the fluid. However, when the egg 5 reaches the most distal part of the elongated body 2, a gap is formed in the aperture 6, as shown in Figure 1, which means that the fluid contained in the lumen 3 can leak out of the lumen through the fluid pathway F, such that the fluid is expelled without exerting a pressure on the egg 5. Therefore, in some cases, the egg 5 may remain attached to the distal tip 4 even after the plunger has been advanced completely and the fluid has been expelled completely. This reduces the change of successful implantation since the egg 5 may move or even become detached from the endometrium upon removing the elongated body 2.

Figure 2 shows a schematic perspective view of an apparatus 10 suitable for delivering a fertilized egg or embryo 50 (referred to hereinafter as "egg" for short) into a maternal uterine endometrium according to one or more embodiments. The apparatus 10 comprises an elongated body 20 comprising a distal tip 40 and a lumen 30 extending therethrough to an aperture 60 at the distal tip 40. The lumen 30 is configured to receive and expel the egg 50 through the aperture 60. The apparatus 10 also comprises a plunger 44 configured to be slidably received in the lumen 30. The distal tip 40 comprises a piercing portion 70 for piercing the maternal uterine endometrium, and the aperture 60 has a concave shape. The piercing portion may have any shape and sharpness suitable to pierce the endometrium. In some embodiments, the piercing portion may comprise a tip or an edge having an edge radius of less than 2 µm, preferably less than 1 µm. The concave shape of the aperture 60 reduces or eliminates the amount of fluid that can leak out of the lumen 30 without exerting pressure on the egg 50 at the distal end of the distal tip 40, such that the egg 50 is less likely to remain at the distal tip 40 when the fluid is expelled completely. This increases the likelihood of a successful implantation.

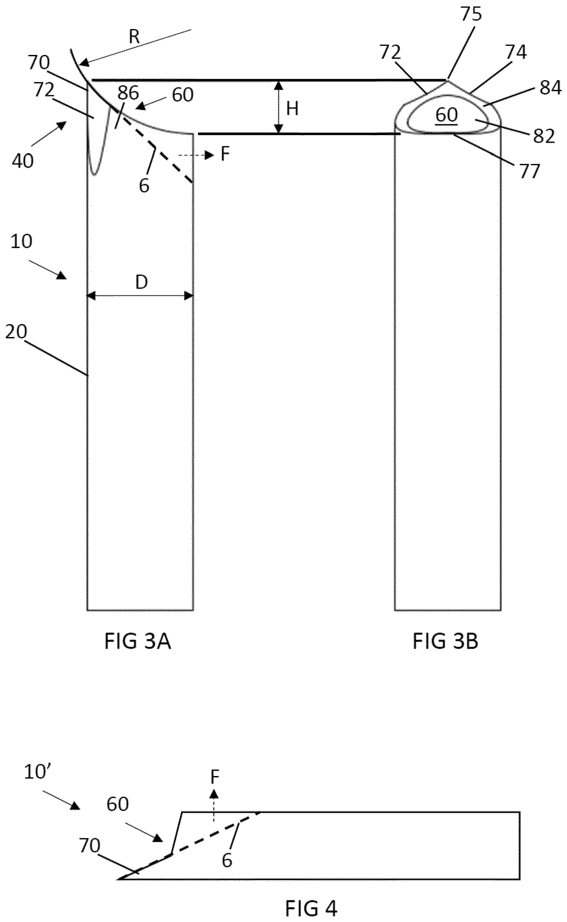

Figures 3A and 3B show different schematic side views of the apparatus 10 shown in Figure 2. In Figure 3A, the concave shape of the aperture 60 is shown with respect to the shape of the aperture 6 of the apparatus 1. It can be seen that the available fluid pathway F shown in Figure 1 is blocked by the concave shape of the aperture 60. As shown in Figure 3B, the piercing portion 70 may comprise a double-bevelled tip, comprising back cut bevels, for piercing the maternal endometrium. In particular, the piercing portion 70 may comprise a first surface 72 and a second surface 74 which are bevelled with respect to the outer surface of the elongated body 20, the bevelled surfaces 72 and 74 forming a sharp tip 75 at the distal end of the distal tip 40.

As shown in Figures 3A and 3B, the distally facing surface 84 of the distal tip 40 which defines the aperture 60 may have a curved concave shape having a radius of curvature R, such that the aperture 60 has a curved concave shape having a radius of curvature R. A curved concave shape having a radius of curvature is preferred because the aperture 60 generally follows the spherical shape of the egg 50, such that when the egg 50 is at the distal end of the distal tip 40, the aperture conforms to the shape of the egg 50 and prevents any fluid pathways F from being produced. In preferred embodiments, the radius of curvature R is between 1.5 and 4 times the outer diameter D of the elongated body 20, which is considered optimal for preventing fluid pathways F from being produced for typical egg and embryo sizes.

As shown in Figure 3A, preferably the curved concave shape extends between the piercing portion 70 and a lowermost portion of the distal tip 40, wherein the lowermost portion of the distal tip 40 extends perpendicularly to the longitudinal axis of the elongated body 20. This ensures that fluid pathways are minimised while at the same time maintaining contact between the distal tip 40 and the egg 50 when it is placed at the distal end of the elongated body 20.

In preferred embodiments, the height of the distal tip 40 as measured from the lowermost portion 77 of the distal tip 40 to the distal end 75 of the piercing portion 70 is between 0.25 and 1.25 times the outer diameter D of the elongated body 20, and more preferably between 0.5 and 1.25 times the outer diameter D of the elongated body 20.

The distal tip can be made of a biocompatible metal, for example, stainless steel.

In preferred embodiments, the distal tip 40 comprises a hydrophobic coating. The hydrophobic coating can be provided on one or more of an inner surface 82 of the distal tip 40, an outer surface 86 of the distal tip 40, and a distally facing surface 84 of the distal tip 40. The hydrophobic coating may comprise or consist of a polymer or polymer composite, preferably any of the following: parylene, acrylic, polyethylene, polyurethane, and polytetrafluoroethylene, and composites thereof. The hydrophobic coating further reduces the likelihood of the egg 50 remaining at the distal tip 40 after the fluid has been expelled.

Figure 4 shows a schematic side view of another apparatus 10' according to one or more embodiments. The apparatus may comprise any of the features disclosed in reference to Figures 2, 3A, and 3B, except the aperture 60 of the apparatus 10' does not comprise a curved concave shape. Instead of the curved concave shape shown in Figures 2, 3A, and 3B, the aperture 60 can adopt a concave shape formed by a plurality of flat surfaces. It will be seen that although the concave shape in the illustrated embodiment is formed by two flat surfaces, it can be formed by any number of a plurality of flat surfaces. The concave shape of the aperture 60 is again shown with respect to the shape of the aperture 6 of the apparatus 1. It can be seen that the available fluid pathway F shown in Figure 1 is blocked by the concave shape of the aperture 60 of the apparatus 10'.

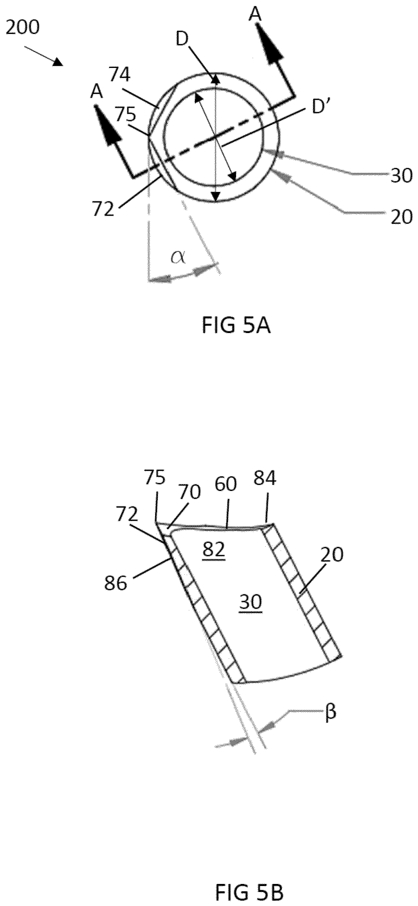

Figure 5A shows a schematic top view of an apparatus 200 according to one or more embodiments. Figure 5B shows a cross-sectional side view of the apparatus 200 shown in Figure 5A taken along line A-A. The apparatus 200 may comprise any of the features of the apparatus described herein (for example, apparatus 10 or 10'). The piercing portion 70 has a first surface 72 and a second surface 74 which are bevelled with respect to the outer surface of the elongated body 20, the bevelled surfaces 72 and 74 forming a sharp tip 75 (a double-bevelled tip) at the distal end of the distal tip 40. The bevelled surface 72 is defined by the bevel angle α in the plane perpendicular to the longitudinal axis of the elongated body 20 and the draft angle β with respect to the longitudinal axis of the elongated body 20. The bevel angle α is defined as the angle formed by the plane containing the bevelled surface 72 with the tangent of the surface of the elongated body 20 at the tip 75. The draft angle β is defined as the angle formed by the plane containing the bevelled surface 72 with the longitudinal axis of the elongated body 20. The bevel angle and the draft angle of the bevelled surface 74 are similarly defined. The bevel angles of the bevelled surfaces 72 and 74 are preferably between 20° and 40°. The draft angles of the bevelled surfaces 72 and 74 are preferably between 1° and 20°. The bevel and draft angles of the bevelled surfaces 72 and 74 can be the same (as illustrated) or different.

**In** preferred embodiments, the diameter of the lumen 30 is between 1 and 2 times the outer diameter of the egg to be implanted, more preferably between 1 and 1.5 times the outer diameter of the egg to be implanted.

In preferred embodiments, the outer diameter of the elongated body 20 is between 1.5 and 3 times the diameter of the lumen 30, more preferably between 1.5 and 2 times the diameter of the lumen 30.

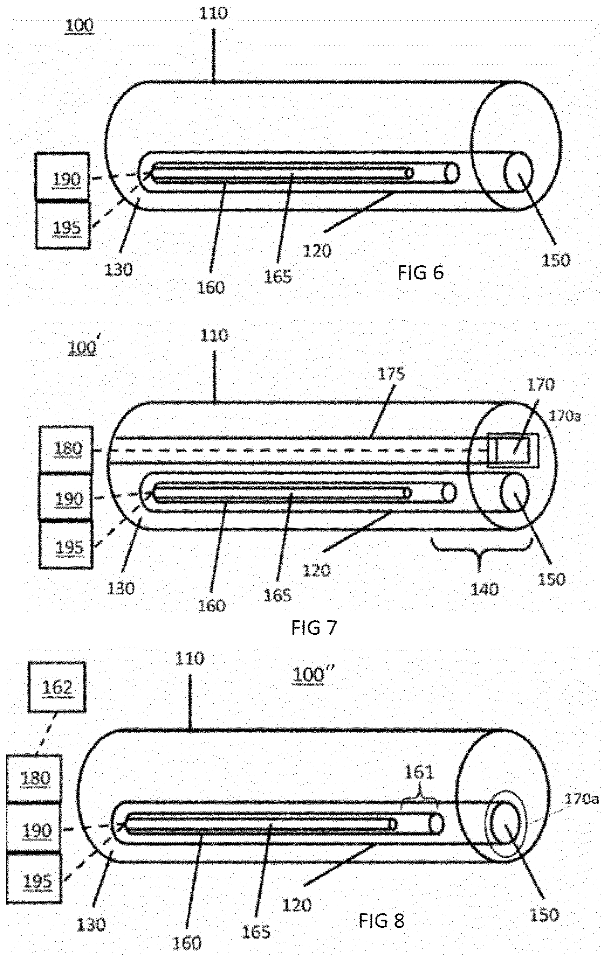

Figure 6 shows a schematic view of an apparatus 100 suitable for delivering a fertilized egg or embryo into a maternal uterine endometrium according to one or more embodiments, wherein the apparatus 100 comprises a body 110 (for example, a catheter, also referred to as outer body) configured to fit within a lumen of the female reproductive system. The body comprises one or more lumens including a lumen 120 extending from a proximal end 130 of the body 110 to a distal portion 140 of the body 110 and having a distal aperture 150 in the distal portion 140 of the body. The lumen 120 is configured to slidably receive an inner body 160 having a distal end suitable for penetrating the endometrial epithelium. The inner body 160 can be any of the elongated bodies 20 disclosed herein and may comprise any of the features of the apparatus disclosed in reference to the preceding figures. The apparatus 100 may also comprise a first actuator 190 which allows advancing the inner body 160 out from the distal aperture 150 of the body 110, and a second actuator 195 which allows expelling a fertilized egg or embryo from the inner body.

The apparatus 100 may comprise a covering layer (not shown) that at least partially covers the distal aperture of one or more of the lumens (for example, in the embodiment illustrated in Figure 6, the covering layer can cover the distal aperture 150 of the lumen 120). The provision of a covering layer prevents mucus and other bodily fluids from entering the lumen 120 as the apparatus advances through the female reproductive system.

The dimensions of the body 110 are enlarged in Figure 6 for the sake of clarity, while in reality the body 110 is a narrow elongated body configured to extend into the uterus until the endometrial epithelium.

As described above, the inner body 160 has a distal end suitable for penetrating the endometrial epithelium (for example, the piercing portion 70). In this embodiment, the inner body 160 is shown located in the lumen 120 of the body 110. A plunger 165, which can be plunger 44, is housed inside the inner body 160 and is configured to advance towards the distal end of the inner body 160 when actuated by the second actuator 195 and to expel a fertilized egg which is located inside the inner body 160. When provided, the covering layer can at least partially cover the distal aperture 150 and the inner body 160 can be configured to pierce through the covering layer when the inner body 160 advances out from the distal aperture of the body, such that the inner body 160 can advance through the covering layer 170a and outwardly from the distal aperture 150 in order to penetrate the endometrial epithelium.

Figure 7 shows a schematic view of another apparatus 100' according to one or more embodiments. The apparatus 100' may comprise any of the features described in reference to Figure 6. The inner body 160 can be any of the apparatus 10, 10' disclosed in reference to Figures 2 to 5B. The apparatus 100' may further comprise a measurement assembly comprising a measurement portion 170 arranged in the distal portion 140 of the body 110 (i.e., proximal to the distal aperture 150). The measurement assembly 170 is configured to measure whether the apparatus is in a first state indicating that the distance between the distal aperture 150 of the body 110 and the endometrial epithelium is greater than a predetermined distance (i.e., in the direction in which the inner body advances from the distal aperture 150), or a second state indicating that the distance between the distal aperture 150 of the body 110 and the endometrial epithelium is equal to or less than a predetermined distance. The apparatus 100' further comprises an indicator device 180 coupled to the measurement assembly 170 through a connection that extends through a lumen 175 and is configured to indicate that the measurement assembly 170 is in the first state or the second state. The measurement assembly can be removed from the body 110. In particular, the measurement portion 170 can be slidably received in a lumen of the body such that it can be extracted from the body 110 through the lumen, which means that the measurement assembly may be reusable. The inner body 160 and the measurement assembly can be received in the same lumen or in different lumens. The apparatus 100' also comprises a first actuator 190 which allows advancing the inner body 160 out from the distal aperture 150 of the body 110 by at least the predetermined distance, and a second actuator 195 which allows expelling a fertilized egg from the inner body (for example, by advancing a plunger 165). As illustrated, a covering layer 170a can at least partially cover one of the distal apertures of the lumens. When the covering layer 170a covers the distal aperture of the lumen 175 and, therefore, the measurement portion 170, the covering layer prevents the measurement portion 170 from being exposed to mucus and increases its reusability when the measurement assembly can be removed from the apparatus (i.e., in the embodiments in which the measurement assembly is not permanently attached to the inner lumen 175 and can be removed).

In some embodiments, the measurement portion 170 is a camera proximal to the aperture 150 and configured to view a portion of the endometrial epithelium.

The measurement assembly may be any suitable measurement assembly which allows determining whether the distance between the distal aperture 150 of the body 110 and the endometrial epithelium is greater or less than the predetermined distance. It can be seen that the measurement assembly can provide a qualitative measurement (for example, the measurement assembly may transition between two states) or a quantitative measurement (for example, actually measuring a parameter which indicates a value of the distance to the endometrial epithelium and comparing the parameter to a threshold value which indicates the predetermined distance). The measurement portion 170 is the part of the measurement assembly which is configured to interact with the endometrial epithelium in order for the measurement assembly to perform the determination and may comprise any suitable element or elements. The elements of the measurement portion 170 can be connected to the components of the proximal end of the apparatus 100, such as the components of the measurement assembly (not shown) and the indicator device 180, through connections extending through one or more lumens 175 in the body 110. For example, the measurement portion may comprise components optically, acoustically, or electrically connected to the proximal components of the measurement assembly, such as light or sound emitters or receivers, electrical signal components, or electrical power sources. The measurement assembly may be any of the measurement assemblies disclosed in PCT application number PCT/EP2021/078712, which is incorporated herein in its entirety by reference.

Figure 8 shows a schematic view of an apparatus 100'' according to one or more embodiments. The apparatus 100" may comprise any of the features of the apparatus disclosed in reference to Figures 6 and 7, and the inner body 160 may comprise any of the features of the elongated bodies 10, 10' disclosed herein. In the embodiment of Figure 8, the measurement assembly 162 comprises a capacitance sensor configured to make electrical contact with a distal portion of the inner body. The measurement assembly 162 is configured to measure whether the apparatus 100 is in a first state indicating that the distance between the distal end of the inner body 160 and the endometrial epithelium is greater than a predetermined distance (i.e., in the direction in which the inner body advances from the distal aperture 150 of the body 110), or a second state indicating that the distance between the distal end of the inner body 160 and the endometrial epithelium is equal to or less than a predetermined distance. In one embodiment, the distal portion 161 of the inner body 160 is electrically conductive, and the measurement assembly 162 is configured to make electrical contact with said distal portion 161 of the inner body 160. The measurement assembly thereby provides an indication of the capacitance. In one embodiment, the first state is indicated by means of a capacitance measurement below a threshold value, and the second state is indicated by means of a capacitance measurement above the threshold value. When provided, the covering layer 170a can cover the distal aperture 150 to prevent mucus from entering the lumen 120 and affecting the capacitance measurements of the distal portion 161 as the inner body 160 is moved towards the distal aperture 150. The inner body 160 is configured to pierce the covering layer 170a such that it can extend distally from the apparatus 100 to penetrate the endometrial epithelium and transfer the fertilized egg.

In a particular embodiment, the capacitance varies according to the variation of the distance between the distal portion 161 of the inner body 160 and the endometrial epithelium, i.e., the capacitance value changes depending on the proximity of the distal portion 161 of the inner body 160 with the endometrial epithelium. In one embodiment, the predetermined distance is zero (which therefore means contact between the distal portion 161 of the inner body 160 and the endometrial epithelium). In one embodiment, the capacitance value is measured by means of the measurement assembly 162, comprising an electrical system which is electrically connected to the distal portion 161 of the inner body 160 for measuring the capacitance.

All of the foregoing falls completely within the scope of the present disclosure and is considered to form the basis for alternative embodiments in which one or more combinations of the above described features are applied, without limitation to the specific combination disclosed above.

In light of the foregoing, there will be many alternatives which implement the teaching of the present disclosure. It is expected that one skilled in the art will be capable of modifying and adapting the above disclosure to suit their own circumstances and requirements within the scope of the present disclosure, while retaining some or all technical effects thereof, either disclosed in or derivable from the above, in light of their common general knowledge in this art. All these equivalents, modifications, or adaptations fall within the scope of the present disclosure.

## Claims

1. An apparatus suitable for delivering a fertilized egg or embryo into a maternal uterine endometrium, comprising:
an elongated body comprising a distal tip and a lumen extending therethrough to an aperture at the distal tip, the lumen being configured to receive and expel the fertilized egg or embryo through the aperture; and
a plunger configured to be slidably received in the lumen;
wherein the distal tip comprises a piercing portion for piercing the maternal uterine endometrium, and the aperture has a concave shape.

2. The apparatus according to claim 1, wherein the piercing portion comprises a double-bevelled tip for piercing the maternal uterine endometrium.

3. The apparatus according to claim 2, wherein the double-bevelled tip comprises two bevelled surfaces having bevel angles between 20° and 40° and a draft angle between 1° and 20°.

4. The apparatus according to any preceding claim, wherein the aperture has a curved concave shape having a radius of curvature.

5. The apparatus according to claim 4, wherein the elongated body has an outer diameter and the curved concave shape of the aperture has a radius of curvature between 1.5 and 4 times the outer diameter of the elongated body.

6. The apparatus according to claim 4 or 5, wherein the curved concave shape extends between the piercing portion and a lowermost portion of the distal tip, wherein the lowermost portion of the distal tip extends perpendicularly to the longitudinal axis of the elongated body.

7. The apparatus according to claim 6, wherein the elongated body has an outer diameter and the height of the distal tip as measured from the lowermost portion to the distal end of the piercing portion is between 0.5 and 1.25 times the outer diameter of the elongated body.

8. The apparatus according to any preceding claim, wherein the distal tip is made of a biocompatible metal, preferably stainless steel.

9. The apparatus according to any preceding claim, wherein the distal tip comprises a hydrophobic coating.

10. The apparatus according to claim 9, wherein the hydrophobic coating is provided on at least one of: an inner surface of the distal tip, an outer surface of the distal tip, and a distally facing surface of the distal tip.

11. The apparatus according to claim 9 or 10, wherein the hydrophobic coating comprises or consists of a polymer or polymer composite, preferably any of the following: parylene, acrylic, polyethylene, polyurethane, and polytetrafluoroethylene, and composites thereof.

12. The apparatus according to any preceding claim, wherein the diameter of the lumen is between 1 and 1.5 times the outer diameter of the egg to be implanted.

13. The apparatus according to any preceding claim, wherein the elongated body is an inner body, the apparatus further comprising an outer body configured to fit within a lumen of the female reproductive system, the outer body comprising:
a lumen extending from a proximal end of the body to a distal portion of the body and having a distal aperture in the distal portion of the body, the lumen slidably receiving the inner body;
the apparatus further comprising:
a first actuator which allows advancing the inner body out from the distal aperture of the body; and
a second actuator which allows advancing the plunger to expel a fertilized egg from the lumen of the inner body.

14. The apparatus according to claim 13, further comprising a measurement assembly, the measurement assembly extending through a lumen of one or more lumens of the outer body and comprising a measurement portion proximal to a distal aperture of the one or more lumens, the measurement portion being configured to measure whether the apparatus is in a first state indicating that a distance between the distal end of the body or the inner body and the endometrial epithelium is greater than a predetermined distance, or a second state indicating that a distance between the distal end of the body or the inner body and the endometrial epithelium is equal to or less than a predetermined distance.

15. The apparatus according to claim 14, wherein the measurement assembly comprises a capacitance sensor configured to make electrical contact with a distal portion of the inner body.
